# EUROPEAN PATENT SPECIFICATION

(11) **EP 4 134 177 B1**
(45) Date of publication and mention of the grant of the patent: **13.03.2024**
(21) Application number: 20936228.4
(22) Date of filing: 22.05.2020
(51) Int. Cl.: B09B 3/00

(54) **APPARAUS FOR CONVERTING WASTE TO BIOGAS AND METHOD FOR CONVERTING WASTE TO BIOGAS**
VORRICHTUNG ZUR UMWANDLUNG VON ABFALL IN BIOGAS UND VERFAHREN ZUR UMWANDLUNG VON ABFALL IN BIOGAS
APPAREIL DE CONVERSION DE DÉCHETS EN BIOGAZ ET PROCÉDÉ DE CONVERSION DE DÉCHETS EN BIOGAZ

(43) Date of publication of application: 15.02.2023
(73) Proprietor: Shinko Tecnos Co.,Ltd., Ichinomiya-shi, Aichi 491-0043 (JP)
(72) Inventor: KIMURA Mamoru, Ichinomiya-shi, Aichi 491-0043 (JP); NAGASAWA Kentaro, Ichinomiya-shi, Aichi 491-0043 (JP)
(74) Representative: Zacco Sweden AB
(86) International application number: PCT/JP2020/020398
(87) International publication number: WO 2021/234971

(56) References cited:
- JP-A- S5 817 895
- JP-A- 2003 094 022
- JP-A- 2004 008 912
- JP-A- 2004 237 238
- JP-A- 2006 239 623
- JP-A- 2011 240 253

## Description

### TECHNICAL FIELD

The present invention relates to a waste gasification treatment device, a waste gasification treatment system, and a waste gasification treatment method.

### BACKGROUND OF THE INVENTION

The invention of the prior art 1 is a method for producing a product by hydrolyzing a raw material containing a plant waste with steam, comprising: a hydrolysis treatment step for hydrolyzing the raw material with steam; a cleaning step for cleaning the hydrolyzed raw material with a cleaning liquid; and a separation step for separating the cleaned raw material into a solid component and a liquid component, wherein at least one of the solid component and the liquid component is used as the product. The method includes a drying step for drying the solid component and a molding step for pelletizing the solid component, and a biomass fuel is produced from the solid component.

The invention of the prior art 2 is a hydrolysis treatment device for hydrolyzing a raw material containing organic waste, the hydrolysis treatment device comprising: a treatment vessel having a raw material charging port and a product discharging port; a stirring means provided inside the treatment vessel for stirring the raw material; a steam supply pipe for supplying steam to the treatment vessel; and a steam exhaust pipe for discharging steam from the treatment vessel. The treatment vessel has a plurality of steam supply ports.

### PRIOR ART

Prior Art 1: JP Pat. No. 6,190,082
Prior Art 2: JP Pat. No. 6,409,237 JP-2011-240253A discloses a further known prior art.

However, in the production method of the product described in the prior art 1 and the hydrolysis treatment device described in the prior art 2, since the solid component or the liquid component obtained by hydrolyzing the waste is utilized, solid-liquid separation is required for obtaining the solid component or the liquid component. In the cases of prior arts 1 and 2, since using of the solid component is mainly aimed, and the drying step of the hydrolysis-produced solid component and the pellet molding step are required, the manufacturing cost of a device for the steps and energy cost are high, the treatment time of the steps becomes long, and a pollution prevention treatment of exhaust gas generated by burning a solid component is required.

This invention aims at omitting a drying step for drying a product produced by hydrolyzing a waste with steam, a pellet molding step, and a treatment step of exhaust gas generated by combustion of pellets, and thus reducing the device manufacturing cost, energy cost, and running cost.

### MEANS FOR SOLVING THE PROBLEM

A waste biogasification treatment device according to the present invention comprises a hydrolysis device comprising: a treatment vessel having a raw material charging port and a product discharging port; a stirring means provided inside the treatment vessel and stirring a raw material; a steam supply pipe for supplying steam to the treatment vessel; and a steam exhaust pipe for discharging steam from the treatment vessel. The waste biogasification treatment device further comprises: a solid-liquid separation device for performing solid-liquid separation of the hydrolyzed raw material to obtain a product having a water content of 70-90%; and a biogasification and recovering device for biogasifying the product and recovering the generated biogas.

A waste biogasification treatment method according to the present invention comprises: a raw material charging step for charging a raw material containing an organic waste into a treatment vessel; a hydrolysis treatment step for subjecting the raw material to hydrolysis treatment with steam; a hydrolysis treatment step for hydrolyzing said raw material with steam; a solid-liquid separation step for performing solid-liquid separation of the raw material hydrolyzed in the hydrolysis treatment step to obtain a product having a water content of 70 -90%; and a biogasification and recovering step for biogasifying the product and recovering generated biogas. In the hydrolysis treatment step, an internal pressure of the treatment vessel is 1.2 MPa to 3.0 MPa, a treatment temperature is 189-234° C, and a retention time after the treatment temperature reaches a target temperature zone is 10 minutes to 2 hours. It is preferable that the method further comprises a cleaning step for cleaning the raw material hydrolyzed in the hydrolysis treatment step before the solid-liquid separation step.

Provided is a waste gasification treatment system comprising a hydrolysis treatment device, a biogasification and recovering device, and a biomass boiler for generating steam using organic matter as a fuel, wherein the fuel of the biomass boiler may be obtained by drying a product produced by hydrolysis treatment in the hydrolysis treatment device to a water content of 20% or less.

The biomass boiler includes a fuel supply port for supplying a fuel, and the fuel supply port may be connected to a discharging port of a drying device for drying the product.

The waste biogasification treatment method according to the present invention comprises: a raw material charging step for bringing a treatment vessel into a sealed state after charging the raw material into the treatment vessel; a pressurizing and temperature raising step for supplying steam to the treatment vessel from a plurality of positions and pressurizing and raising the temperature; a temperature retaining step for holding the temperature after the inside of the treatment vessel reaches a target temperature zone; a steam exhaust step for discharging steam inside the treatment vessel after the pressurizing and temperature raising step and a temperature retaining step; a product discharging step for discharging the raw material subjected to the hydrolysis treatment inside the treatment vessel as a product; and a biogasification and recovering step for biogasifying the product and recovering the generated biogas.

It is preferable that the raw material in the treatment vessel is stirred by a stirring means when the raw material is charged into the treatment vessel, when the temperature inside the treatment vessel is retained, and when the temperature inside the treatment vessel is retained after the temperature of the treatment vessel reaches the target temperature zone.

### ADVANTAGEOUS EFFECT OF THE INVENTION

According to the present invention, various costs such as a device manufacturing cost, an energy cost, and a running cost can be reduced by omitting a drying step of a product produced by hydrolyzing a waste with steam, a pellet molding step, and a treatment of exhaust gas generated by combustion of a pellet. Power generation can be performed by the generated biogas. The decomposition temperature at the time of hydrolysis is low and the temperature holding time (decomposition time) is prolonged, so that the carbonization of the waste which is a hindrance to the biogasification can be reduced.

### BRIEF DESCRIPTION OF THE DRAWINGS

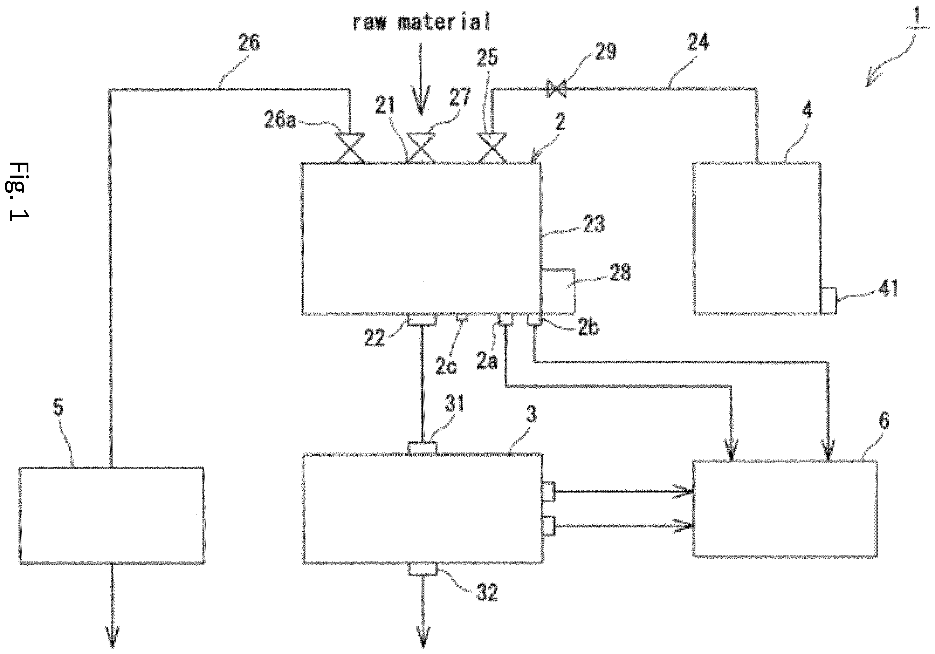

FIG. 1 is a block diagram of a waste biogasification treatment device according to an embodiment of the present invention.
FIG. 2 is a flowchart of a waste biogasification treatment method according to an embodiment of the present invention.

### DETAILED DESCRIPTION OF THE INVENTION

Embodiments of the present invention will now be described in detail with reference to the drawings.

A waste biogasification treatment device 1 according to an embodiment of the present invention is provided with a hydrolysis device 2 for hydrolyzing a raw material containing organic waste with steam to produce a product, a biogasification and recovering device 3 for receiving the product produced by decomposition in the hydrolysis device 2 and biogasifying the product to recover the generated biogas, a boiler 4 for supplying steam to the hydrolysis device 2, a condenser 5 for removing the steam pressure of the hydrolysis device 2, and a control device 6 for controlling the pressure and temperature and the like of the hydrolysis device 2. The device is provided with a water content adjusting device, a cleaning device (not shown) for cleaning the hydrolyzed raw material with a cleaning liquid, and/or a solid-liquid separating device (not shown) for separating the cleaned raw material into a solid component and a liquid component as necessary depending on various circumstances such as raw materials.

The hydrolysis device 2 hydrolyzes a raw material to produce a product to be subjected to a biogasification treatment, and comprises: a treatment vessel 23 having a raw material charging port 21 and a product discharging port 22; a stirring means provided inside the treatment vessel 23 and for stirring the raw material; a steam supply pipe 24 for supplying steam to the treatment vessel 23; a plurality of steam supply ports 25 for supplying the steam from the steam supply pipe 24 to the treatment vessel 23; and a steam exhaust pipe 26 for discharging the steam from the treatment vessel 23.

The treatment vessel 23 is horizontal but may be vertical. The raw material to be subjected to the waste biogasification treatment contains an organic waste, such as fishery processed product residues, various kinds of sludge, agricultural products, agricultural product processed product residues, beverage residue, municipal waste, and the like. The raw material is preferably crushed to a size of 100 mm or less by using a crushing means (not shown). The crushing means may be a well-known one, but may be a uniaxial rotary crushing means when a soft material such as a fishery processed product residue is used as a raw material.

The crushed raw material may be measured in an amount corresponding to the inner volume of the treatment vessel 23 of the waste biogasification treatment device 1 and temporarily stored in a raw material charging silo (not shown). The inner volume of the raw material charging silo (not shown) depends on the inner volume of the treatment vessel 23 and is usually 1.1 to 1.2 times the inner volume of the treatment vessel 23. When storing a raw material containing acidic or basic substances such as fishery processed product residue, the raw material charging silo may be formed of a material having corrosion resistance.

Since high pressure steam is supplied to the inside of the treatment vessel 23 from a steam supply port 25 described later, the treatment vessel 23 needs to satisfy the requirement of the class-1 pressure vessel fixed by Japanese law. Therefore, the inner volume, the diameter, the plate thickness, and the material of the treatment vessel 23 are designed within the requirements of the class-1 pressure vessel according to the amount of the raw material.

Although the higher pressure-resistant performance of the treatment vessel 23 may be better, the pressure resistance is usually 2 MPa to 10 MPa and preferably 3 MPa to 8 MPa in consideration of practicality and economy.

The hydrolysis device 2 often hydrolyzes the raw material under high temperature, high pressure and strong acid or strong basic conditions. The treatment vessel 23 may be formed by lining the inside of the pressure-resistant structure with a thin plate of a chemical resistant stainless steel (SUS316), a fluorine-based or a silicone-based polymer material. Conventional stainless steel (SUS304) may be used depending on the raw material to be treated.

The raw material charging port 21 is formed on the upper part of the treatment vessel 23. When a raw material charging port 21 is formed on the side surface of the treatment vessel 23, the stirring means described later becomes an obstacle, and the charging of the raw material becomes difficult.

The raw material charging port 21 is connected to an openable and closable pressure resistant valve 27. Since the raw material charging port 21 is closed during the hydrolysis treatment, the pressure resistance of the pressure resistant valve 27 is preferably equal to or higher than the pressure resistance of the treatment vessel 23.

The product discharging port 22 is formed at a lower part of the treatment vessel 23.

The treatment vessel 23 has a stirring means (not shown) driven by a motor 28 in order to stir and mix the internal raw material. A paddle type or screw type well-known stirrer can be used as the stirring means. The stirring means can crush and mix the raw material which has high viscosity and high weight inside the treatment vessel 23. The raw material can be brought into a uniform state.

When the stirring means (not shown) is of a screw type, and the treatment vessel 23 has an inner diameter of 100 cm and a total length of 250 cm, a stirring blade preferably has an outer diameter of 95 cm to 98 cm, a pitch of 30 cm to 50 cm, and is in a shape of a fan or a paddle.

The rotational speed of the stirring means is suitably set according to the raw material in a range of 0.1 rpm to 50 rpm, preferably 0.5 rpm to 30 rpm. Since the stirring means (not shown) performs crushing and shearing simultaneously with stirring and mixing, the stirring torque is more important than the stirring speed.

The stirring by the stirring means (not shown) is preferably started simultaneously with the charging of the raw material. The stirring condition is determined according to the reaction time of the waste biogasification treatment device 1 and the state of the raw material. The start time and completion time is appropriately set in response to the reaction time and the state of raw material. The stirring operation may be either continuous or intermittent. The stirring operation may be controlled so that the rotation direction is controlled with a timer so as to repeat normal rotation and reverse rotation every predetermined time. When the stirring means is rotated only in a fixed direction, the raw material may be biased to one side, and the crushing and mixing by stirring cannot be uniformly performed.

A structure for supplying steam to the inside of the treatment vessel 23 and exhausting the steam from the treatment vessel 23 is described. The treatment vessel 23 has a plurality of steam supply ports 25 along the longitudinal direction of the treatment vessel 23. The plurality of steam supply ports 25 are connected to a plurality of branch pipes (not shown) branched from a steam supply pipe 24 downstream of a control valve 29.

The control valve 29 controls the pressure inside the treatment vessel 23 to a prescribed pressure, for example, 3 MPa by a pressure gauge 2a provided in the treatment vessel 23. The temperature inside the treatment vessel 23 is measured by a thermometer 2b. A safety valve 2c is provided on the treatment vessel 23 for cases when meters and gauges such as the pressure gauge 2a and various valves are broken down.

The boiler 4 is supplied with fuel from a fuel supply port 41. The capacity of the boiler 4 is set according to the internal volume and temperature raising time of the treatment vessel 23.

The steam supply pipe 24 is connected to the boiler 4 through the control valve 29. The boiler 4 generates steam using organic matter as fuel, and has the ability to adjust the treatment vessel 23 to a predetermined pressure (steam pressure) and temperature.

The exhaust of the steam from the treatment vessel 23 may be carried out to a condenser tank filled with water of 3 to 10 times the inner volume of the treatment vessel 23 without using a condenser 5. The condenser tank also functions as a pressure releasing means for decompressing the discharged steam (for example, 3.0 MPa) to nearly a normal pressure (0.1 MPa).

Since the steam can be uniformly supplied to the inside of the treatment vessel 23, the temperature and pressure inside the treatment vessel 23 can be made uniform. By making the temperature and pressure inside the treatment vessel 23 uniform, the hydrolysis treatment to the raw material containing the organic waste can be uniformly advanced, and the product can be made homogeneous.

The boiler 4 may be provided exclusively as in the present embodiment. However, if there is a surplus of steam in a boiler for a factory, and if the steam meets the use condition for the treatment vessel 23, this steam may be directly introduced instead of the steam of the boiler 4. Various types of boilers such as a once-through boiler may be installed together with the boiler 4.

The treatment vessel 23 has a plurality of steam exhaust ports 26a along the longitudinal direction of the treatment vessel 23. The plurality of steam exhaust ports 26a are connected to a plurality of branch pipes (not shown) each having a ball valve (not shown), and are gathered into a steam exhaust pipe 26.

The plurality of ball valves (not shown) adjust the flow quantity or flow rate of the steam from the treatment vessel 23, and the opening degrees are respectively set automatically or manually. The exhaust of the steam from the treatment vessel 23 is carried out by spending, for example, 5 to 60 minutes depending on the internal pressure, and the inside of the treatment vessel 23 is decompressed.

The condenser 5 is a cyclone for recovering aggregates (aggregated liquid and dust) from the discharged steam. The condenser 5 may be a tangential cyclone having a simple structure, an axial flow cyclone, or a multiclone having multiple cyclones connected in parallel or in series. When the outer periphery of the condenser 5 is heated, the flocculated liquid is almost vaporized, and the dust (powdery solid) is recovered from the lower part of the condenser 5.

The condenser 5 also functions as a pressure releasing means for reducing the pressure of the discharged steam (for example, 3.0 MPa) to about 0.5 MPa in addition to recover of the aggregate. Therefore, the internal volume of the condenser 5 is 0.5 -1.5 times the internal volume of the treatment vessel 23.

The steam from which the aggregate is recovered by the condenser 5 is released to the atmosphere. If necessary, a deodorizer is provided on the downstream side of the condenser 5, and the exhausted steam may be deodorized and discharged to the atmosphere. A well-known adsorbent type can be used as the deodorizer, and the adsorbent may be suitably selected from activated carbon, lime, zeolite, etc., according to the component of the odor.

The biogasification and recovering device 3 includes a product receiving port 31 and a biogas discharging port 32 for discharging the generated biogas.

The biogas generated by the biogasification and recovering device 3 is used for (1) a fuel gas for a gas electric power generator and (2) a fuel gas for a burner used in a boiler. Steam may be generated by using heat generated during power generation by the gas electric power generator, and used for hydrolysis.

A method for producing a product according to an embodiment of the present invention is herein described. This method hydrolyzes a raw material containing organic waste to produce a product. This method comprises: a hydrolysis treatment step in which the raw material is hydrolyzed by steam; and a biogasification and recovering step in which the product produced by hydrolysis treatment is biogasified and recovered. If necessary, the method includes: a cleaning step for cleaning the hydrolyzed raw material with a cleaning liquid; and a solid-liquid separation step for separating the cleaned raw material into a solid component and a liquid component and setting the water content of the solid component to, for example, 80%. The solid component is the product. Since the liquid is not an object of the present invention, explanation is omitted.

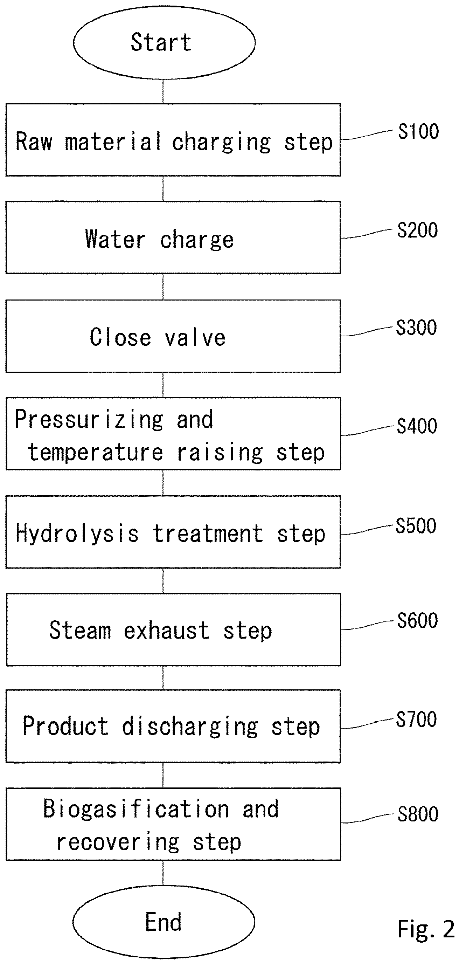

A method for producing a product according to an embodiment of the present invention is herein described with reference to FIG. 2.

### (A raw material charging step S100)

The raw material has a water content (based on mass: hereinafter the same). The proper range of the water content is 40 to 80%, preferably 40 to 70%, especially preferably 40 to 60%. Normally, when the water content of the raw material is less than 40%, water is added, and when more than 80%, the water content is reduced. When the water content is lower than 40%, water, wastewater generated during hydrolyzing, or concentrated water generated at the time of steam exhaust is charged into the treatment vessel 23, and the water content is set to be within the proper range (S200). When the water content exceeds 80% and the water content is reduced, a water content adjusting material may be simultaneously charged from the raw material charging port 21 together with the raw material in the treatment vessel 23. As the water content adjustment material, it is preferable to use an adsorbent having an ability of adsorbing harmful heavy metal ions or the like eluted by hydrolysis treatment together with water absorption ability. The adsorbent is preferably an organic adsorbent. In the case of the organic adsorbent, industrial waste can be utilized and the organic adsorbent itself can also be subjected to hydrolysis treatment. Sawdust, chaff, wood chips, straw, rice straw, etc., may be used as the organic adsorbent. When an inorganic adsorbent is used, slaked lime, zeolite, pumice, etc., may be used. The water content of the water content adjusting material is 30% or less, preferably 15% or less.

The raw material is charged into a treatment vessel of a hydrolysis treatment device.

When the raw material is charged into the treatment vessel, the raw material may be stirred by the rotation of the stirring means in the treatment vessel. The operation of the stirring means may be either continuous or intermittent, but the rotation direction may be controlled by a timer so as to repeat normal rotation and reverse rotation every predetermined time.

After the raw material and the water content adjusting material are charged from the raw material charging port 21 of the treatment vessel 23, the pressure resistant valve 27 on the raw material charging port 21 side is closed, and the treatment vessel 23 is brought into a sealed state (S300).

### (pressurizing and temperature raising step S400)

When the treatment vessel 23 is brought into a sealed state, steam is supplied from the boiler 4 to the inside of the treatment vessel 23 through a steam supply pipe 24 and a control valve 29, and the inside of the treatment vessel 23 is pressurized and heated to a prescribed pressure and temperature.

The internal pressure of the treatment vessel at this time is 1.2 MPa to 3.0 MPa, preferably 1.5 MPa to 2.5 MPa, and the internal temperature is 189 to 234° C, preferably 200° C to 224° C. The temperature retention time for hydrolysis is 10 minutes to 2 hours, preferably 30 minutes to 1.5 hours after the internal temperature reaches the target temperature zone. The target temperature zone is different depending on the kind of the raw material. Since it is saturated steam, when one of pressure and temperature is known, the other is determined. Since the temperature is set relatively low and the retention time is set long, the solid component of the product is not carbonized, and the biogasification can be performed smoothly in the biogasification and recovering step described later. An upper limit is provided for the retention time considering the production capacity. The biogas often contains methane.

### (The hydrolysis treatment step S500)

While the inside of the treatment vessel 23 is maintained at a prescribed pressure and temperature, the raw material, which is an organic waste, starts to be hydrolysis treated. When a prescribed time from the start of the supply of the steam within the retention time passes, the hydrolysis treatment of the raw material is almost completed. The supply of the steam to the inside of the treatment vessel 23 from the boiler 4 is stopped and the hydrolysis treatment is finished. In the hydrolysis treatment step, the raw material is subjected to hydrolysis treatment. When the water content adjustment material is an organic material, the water content adjustment material is also subjected to hydrolysis treatment. Harmful heavy metal ions or the like are eluted into the liquid component and adsorbed by the adsorbent which has not been hydrolyzed, and moved to the solid component.

In the hydrolysis treatment step, the motor 28 may be driven to rotate the stirring means and thus to stir the raw material in the same manner as during the raw material charging step. The stirring operation may be either continuous or intermittent, but the rotation direction may be controlled by a timer so as to repeat normal rotation and reverse rotation every predetermined time.

The time of the hydrolysis treatment is suitably changed according to the treatment amount, etc., but about 10 minutes to about 2 hours is sufficient.

As the raw material is subjected to hydrolysis treatment, a polymer such as cellulose is decomposed into low molecular molecules. That is, plant fibers (cell walls and cell membranes of plants) are made brittle or destroyed. The elements such as potassium, chlorine in the plant cells are eluted or easily eluted into the liquid component.

### (Steam exhaust step S600)

After the completion of the hydrolysis treatment step, the pressure inside the treatment vessel 23 is released to nearly a normal pressure (0.1 MPa) by discharging steam through the steam exhaust pipe 26 connected to the steam exhaust port 26a during 5 minutes to 60 minutes. The steam to be exhausted also includes the dust of the raw material as entrainment.

Preferably, after the completion of the hydrolysis treatment step, the pressure inside the treatment vessel 23 is released for 30 minutes to 60 minutes to a normal pressure of 0.1 MPa to 0.3 MPa by using the condenser 5.

### (A product discharging step S700)

The product (sludge state) produced by hydrolysis treatment in the waste biogasification treatment device 1 is discharged from a product discharging port 22 at the lower part of the treatment vessel 23.

If necessary, a cleaning step and/or a solid-liquid separation step may be performed before the sludge, which is the product of hydrolysis, is subjected to the biogasification treatment.

In the cleaning step, the discharged product is charged into a cleaning device equipped with an agitator, immersed in the cleaning liquid, stirred and cleaned. The cleaning liquid may be water such as factory water or tap water, and the cleaning liquid of preferably 5 times or more, preferably 10 to 20 times the volume of the raw material is used. The temperature of the cleaning liquid may be between about 60° C and a room temperature. The cleaning time is suitably changed according to the amount of treatment, etc., and 15 to 30 minutes is sufficient.

Depending on the state of the product, solid-liquid separation (dehydration) may be or may not be performed by using the solid-liquid separation device before gasification. The case that solid-liquid separation is not performed is the case that the raw material less contains a component such as chlorine and potassium that inhibits methane fermentation in the biogasification and recovering step described later. The case that solid-liquid separation is performed is the case that a large amount of inhibiting components are included, and the product is washed with water in the cleaning step and subjected to solid-liquid separation. Before solid-liquid separation, a cleaning liquid such as water is added to the product after hydrolysis, and the product is stirred and subjected to solid-liquid separation. Thus, the inhibitory component can be further transferred to the liquid side and can be removed. The water content is made to be 70 - 90% by the solid-liquid separation step.

In the case of performing solid-liquid separation, the solid and liquid may be separated by using a solid-liquid separation means such as levitation or sedimentation separation, filter press or centrifugal separation to reduce water content. Depending on the raw material to be hydrolyzed, the solid component of the separated product can be used as a fertilizer or compost, and the liquid component of the product can be used as a raw material for a liquid fertilizer. The water content is made 70 -90% by the cleaning step.

When solid-liquid separation is performed, the liquid component may be roughly removed by a simple filter such as a metal mesh before the product is sent to a solid-liquid separation device for solid-liquid separation. As a result, the product is separated (dehydrated) into a solid component and a liquid component. A solid-liquid separation device such as a screw conveyor type device (for example, see Japanese Published Patent Application No. 2012 -153790) which wrings (dehydrates) the liquid component out of the solid component is used. Since the solid-liquid separation can be continuously performed when using the screw conveyor type device, productivity is improved. A specific step, for example, for the cases when the raw material is EFB, is to be referred to the prior art 1.

The product produced by hydrolysis treatment in the hydrolysis device 2 is charged into a biogasification and recovering device 3.

### (A biogasification and recovering step S800 )

The product is biogasified and the biogas is recovered by a biogasification and recovering device 3. Since the product is hydrolyzed in advance and is primarily decomposed, many biogas can be generated in a short time. The produced biogas is mainly methane and can be used for power generation or as a gas fuel. A commercially available methane fermentation biogas generator may be used as the biogasification and recovering device.

The waste may be treated to reduce the content of chlorine, potassium or the like in the solid component depending on the type of the waste. After the solid-liquid separation step, the step is returned to the cleaning step, and the separation step and the cleaning step are repeated a plurality of times. As a result, the product can be reliably cleaned. Chlorine, potassium or the like in the solid component is eluted into the cleaning liquid by a plurality of times of cleaning, so that the content of chlorine and potassium in the solid component can be reduced. The treatment for lowering the content of potassium in the solid component may be carried out before the raw material charging step depending on the type of waste.

In the present embodiment, the cleaning liquid in the cleaning step is water. Since a special chemical solution is not used as the cleaning liquid for the product, inexpensive factory water or tap water can be utilized.

Although the preferred embodiment of the present invention has been detailed above, the present invention is not limited to the embodiments described above, and various modifications and changes are possible within the scope of the invention as set forth in the claims.

- 1: Waste biogasification treatment device
- 2: Hydrolysis device
- 2a: Pressure gauge
- 2b: Thermometer
- 2c: Safety valve
- 3: Biogasification and recovering device
- 4: Boiler
- 5: Condenser
- 6: Control device
- 14: Steam exhaust port
- 21: Raw material charging port
- 22: Product discharging Port
- 23: Treatment vessel
- 24: Steam supply pipe
- 25: Steam supply port
- 26: Steam exhaust pipe
- 26a: Steam exhaust port
- 27: Pressure resistant valve
- 28: Motor
- 29: Control valve
- 31: Product receiving port
- 32: Biogas discharging port
- 41: Fuel supply port

## Claims

1. A waste biogasification treatment device comprising:
a hydrolysis device comprising: a treatment vessel having a raw material charging port and a product discharging port; a stirring means which is provided inside said treatment vessel and stirs a raw material; a steam supply pipe for supplying steam to said treatment vessel; and a steam exhaust pipe for discharging steam from said treatment vessel;
a solid-liquid separation device for performing solid-liquid separation of said raw material hydrolyzed by said hydrolysis device to obtain a product having a water content of 70 -90%; and
a biogasification and recovering device for biogasifying said product and recovering generated biogas.

2. A waste biogasification treatment method comprising:
a raw material charging step for charging a raw material containing an organic waste into a treatment vessel;
a hydrolysis treatment step for hydrolyzing said raw material with steam;
a solid-liquid separation step for performing solid-liquid separation of said raw material hydrolyzed in said hydrolysis treatment step to obtain a product having a water content of 70 -90%; and
a biogasification and recovering step for biogasifying said product and recovering generated biogas,
wherein in said hydrolysis treatment step, an internal pressure of said treatment vessel is 1.2 MPa to 3.0 MPa, a treatment temperature is 189 to 234° C, and a retention time after said treatment temperature reaches a target temperature zone is 10 minutes to 2 hours.

3. A waste biogasification treatment method according to claim 2, further comprising a cleaning step for cleaning said raw material hydrolyzed in said hydrolysis treatment step before said solid-liquid separation step.

## Patentansprüche

1. Aufbereitungsvorrichtung zur Biogasbildung aus Abfall, umfassend:
Hydrolysevorrichtung, umfassend: ein Aufbereitungsgefäß, das einen Aufnahmeanschluss für Rohmaterial und einen Abgabeanschluss für Produkt; ein Rührmittel, das innen in dem Aufbereitungsgefäß bereitgestellt ist und ein Rohmaterial umrührt; eine Dampfversorgungsleitung zur Versorgung des Aufbereitungsgefäßes mit Dampf und eine Dampfabgasleitung zum Ableiten von Dampf aus dem Aufbereitungsgefäß aufweist;
eine Feststoff/Flüssigkeit-Trennvorrichtung zum Durchführen einer Feststoff/Flüssigkeit-Trennung des Rohmaterials, das durch die Hydrolysevorrichtung hydrolysiert wird, um ein Produkt zu erhalten, das einen Wassergehalt von 70-90 % aufweist; und
eine Biogasbildungs- und Rückgewinnungsvorrichtung zum Biogasbilden des Produkts und Rückgewinnen generierter Biogase.

2. Aufbereitungsverfahren zur Biogasbildung aus Abfall, wobei das Verfahren Folgendes umfasst:
einen Rohmaterialaufnahmeschritt zur Aufnahme eines Rohmaterials, das einen organischen Abfall beinhaltet, in ein Aufbereitungsgefäß;
einen Hydrolyseaufbereitungsschritt zum Hydrolysieren des Rohmaterials mit Dampf;
einen Feststoff/Flüssigkeit-Trennungsschritt zum Durchführen von Feststoff/Flüssigkeit-Trennung des Rohmaterials, das in dem Hydrolyseaufbereitungsschritt hydrolysiert wird, um ein Produkt zu erhalten, das einen Wassergehalt von 70-90 % aufweist; und
einen Biogasbildungs- und Rückgewinnungsschritt zum Biogasbilden des Produkts und Rückgewinnen generierter Biogase,
wobei in dem Hydrolyseaufbereitungsschritt ein Innendruck des Aufbereitungsgefäßes 1,2 MPa bis 3,0 MPa, eine Aufbereitungstemperatur 189 bis 234 C und eine Retentionszeit, nachdem die Aufbereitungstemperatur einen Zieltemperaturbereich erreicht hat, 10 Minuten bis 2 Stunden beträgt.

3. Aufbereitungsverfahren zur Biogasbildung aus Abfall nach Anspruch 2, ferner umfassend einen Reinigungsschritt zum Reinigen des Rohmaterials, das in dem Hydrolyseaufbereitungsschritt vor dem Feststoff/Flüssigkeit-Trennungsschritt hydrolysiert wurde.

## Revendications

1. Dispositif de traitement par biogazéification de déchets comprenant :
un dispositif d'hydrolyse comprenant : un récipient de traitement ayant un port de chargement de matière première et un port de décharge de produit ; un moyen d'agitation qui est disposé à l'intérieur dudit récipient de traitement et agite une matière première ; un tuyau d'alimentation en vapeur pour fournir de la vapeur audit récipient de traitement ; et un tuyau d'échappement de vapeur pour évacuer de la vapeur dudit récipient de traitement ;
un dispositif de séparation solide-liquide pour effectuer une séparation solide-liquide de ladite matière première hydrolysée par ledit dispositif d'hydrolyse pour obtenir un produit ayant une teneur en eau de 70 à 90 % ; et
un dispositif de biogazéification et de récupération pour biogazer ledit produit et récupérer le biogaz généré.

2. Procédé de traitement par biogazéification de déchets comprenant :
une étape de chargement de matière première pour charger une matière première contenant un déchet organique dans un récipient de traitement ;
une étape de traitement par hydrolyse pour hydrolyser ladite matière première avec de la vapeur ;
une étape de séparation solide-liquide pour effectuer une séparation solide-liquide de ladite matière première hydrolysée dans ladite étape de traitement d'hydrolyse pour obtenir un produit ayant une teneur en eau de 70 à 90 % ; et
une étape de biogazéification et de récupération pour biogazer ledit produit et récupérer le biogaz généré,
dans lequel, dans ladite étape de traitement par hydrolyse, une pression interne dudit récipient de traitement est de 1,2 MPa à 3,0 MPa, une température de traitement est de 189 à 234 °C, et un temps de rétention après que ladite température de traitement atteint une zone de température cible est de 10 minutes à 2 heures.

3. Procédé de traitement par biogazéification de déchets selon la revendication 2, comprenant en outre une étape de nettoyage pour nettoyer ladite matière première hydrolysée dans ladite étape de traitement par hydrolyse avant ladite étape de séparation solide-liquide.
